# FASCICULE DE BREVET EUROPEEN

(11) **EP 1 023 287 B1**
(45) Date de publication et mention de la délivrance du brevet: **12.06.2002**
(21) Numéro de dépôt: 98949061.0
(22) Date de dépôt: 14.10.1998
(51) Int. Cl.: C07D 403/12, A61K 31/53, C07D 253/06, C07D 405/12, C07D 471/04

(54) **DERIVES DE LA 3-OXO-2(H)-1,2,4-TRIAZINE EN TANT QUE LIGANDS DES RECEPTEURS 5HT1A**
3-OXO-2(H)-1,2,4-TRIAZINDERIVATE ALS 5HT1A REZEPTOR LIGANDEN
3-OXO-2(H)-1,2,4-TRIAZINE DERIVATIVES AS LIGANDS OF 5HT1A RECEPTORS

(30) Priorité: 16.10.1997 FR 9712955
(43) Date de publication de la demande: 02.08.2000
(73) Titulaire: PIERRE FABRE MEDICAMENT, 92100 Boulogne-Billancourt (FR)
(72) Inventeur: PATOISEAU, Jean-François, F-81100 Castres (FR); DUPONT-PASSELAIGUE, Elisabeth, F-81100 Castres (FR); KOEK, Wouter, F-81290 Viviers-les-Montagnes (FR)
(74) Mandataire: Ahner, Francis
(86) Numéro de dépôt international: FR9802205
(87) Numéro de publication internationale: WO9920622

(56) Documents cités:
- WO-A-95/01965
- WO-A-96/16949

## Description

La présente invention a pour objet de nouveaux dérivés de la 3-oxo-*(2H)*-1,2,4-triazine fonctionnalisée en position 5, leur préparation et leur application on thérapeutique.

Les récepteurs 5-HT_{1A} ont été revendiqués pour leur rôle dans différentes pathologies telles que l'hypertension, le dysfonctionnement sexuel, l'anorexie, la mémoire. La principale cible suggérant l'implication des récepteurs 5-HT_{1A} est cependant constituée par les troubles du système nerveux central tels que l'anxiété et la dépression. Les hypothèses, appuyées par des tests sur modèles animaux et des études cliniques suggèrent que des traitements plus efficaces de ces pathologies peuvent être envisagés avec des composés agonistes 5-HT_{1A} de forte affinité et possédant une grande sélectivité et une forte efficacité.

Des dérivés de la 3,5-dioxo-*(2H,4H)*-1,2,4-triazine et de la 3,5-dioxo-6-amino *(2H, 4H)*-1,2,4-triazine ont été revendiqués précédemment par la demanderesse (FR.2.707.294 du 06/07/93 et FR.2.727.682 du 02/12/94).

Les composés de la présente invention se caractérisent par leur puissante affinité vis-à-vis du récepteur 5-HT_{1A} associée à une grande sélectivité, notamment vis-à-vis des récepteurs D₂ et α₁, et une forte activité intrinsèque.

Les composés de l'invention correspondent à la formule générale I 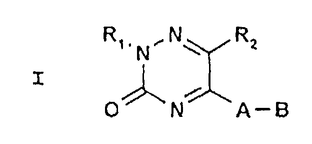 dans laquelle
- R₁ représente :
   - l'hydrogène, uniquement lorsque A est un atome d'azote éventuellement substitué
   - un groupe alcoyle en C₁-C₄ linéaire ou ramifié
   - un groupe phénylalcoyle en C₁-C₄, le noyau phényle étant éventuellement substitué par un ou plusieurs groupements alcoyle en C₁-C₄, alcoxy en C₁-C₃, halogène, trifluorométhyle.
- R₂ représente :
   - l'hydrogène
   - un radical alcoyle en C₁-C₄ linéaire ou ramifié
   - un groupement phényle ou phénylalcoyle en C₁-C₄, le noyau phényle étant éventuellement substitué par un ou plusieurs groupements alcoyle en C₁-C₄, alcoxy en C₁-C₃, halogène, trifluorométhyle.
- A représente un atome d'oxygène ou un atome d'azote éventuellement substitué NR₃.
- R₃ représente l'hydrogène ou un groupe méthyle.
- B représente un groupement de type
   - 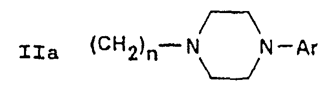 dans lequel Ar représente lui-même une structure aromatique phényle, pyridyle ou pyrimidyle, éventuellement substituée par un ou plusieurs groupements alcoyle en C₁-C₃, alcoxy en C₁-C₃, hydroxy, trifluorométhyle ou halogène et n peut prendre les valeurs entières de 3 à 5.
   - 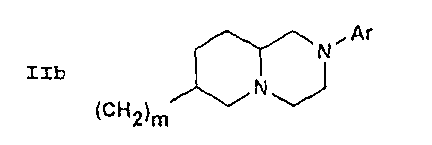 dans lequel Ar est tel que défini dans la formule IIa et m peut prendre les valeurs entières 1 et 2
   - 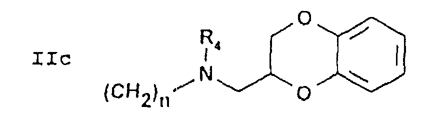
   dans lequel R₄ représente l'hydrogène ou un groupement alcoyle en C₁-C₃ et n peut prendre les valeurs entières de 3 à 5.

L'invention couvre les sels minéraux ou organiques de composés de formule générale I avec les acides pharmaceutiquement acceptables.

Elle couvre, en outre, les différents énantiomères et diastéréoisomères des composés qui possèdent un ou plusieurs carbones asymétriques, ainsi que leurs mélanges en toutes proportions incluant notamment les mélanges racémiques.

### Synthèse

Les composés de la présente invention peuvent être préparés selon diverses méthodes. Ils peuvent être synthétisés en utilisant les voies de synthèse décrites ci-dessous ou en utilisant des méthodes de synthèse connues de l'homme de métier.

La synthèse des composés de formule générale I est caractérisée en ce que l'on condense un dérivé de formule générale III (schéma 1) 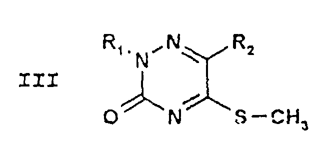 avec un alcool B-OH IV ou une amine BNHR₃ V, R₁, R₂, R₃ et B ayant la même signification que précédemment dans la formule générale I.

Les composés III sont obtenus selon un procédé (schéma 1) caractérisé par les étapes suivantes :
*1* - Condensation de l'acide glyoxylique sur le thiosemicarbazide, suivie d'un traitement basique tel que la soude,
*2* - Méthylation par l'iodure de méthyle en milieu basique aqueux suivi d'un traitement acide tel que l'acide chlorhydrique,
*3* - Sulfuration de la position 5 en présence du réactif de Lawesson dans un solvant tel que la pyridine,
*4* - Méthylation par l'iodure de méthyle en milieu basique aqueux tel que la soude,
*5* - Alkylation de la position 2 par un halogénure d'alcoyle R₁X en présence de NaH dans le DMF, X représentant Cl, Br ou I.

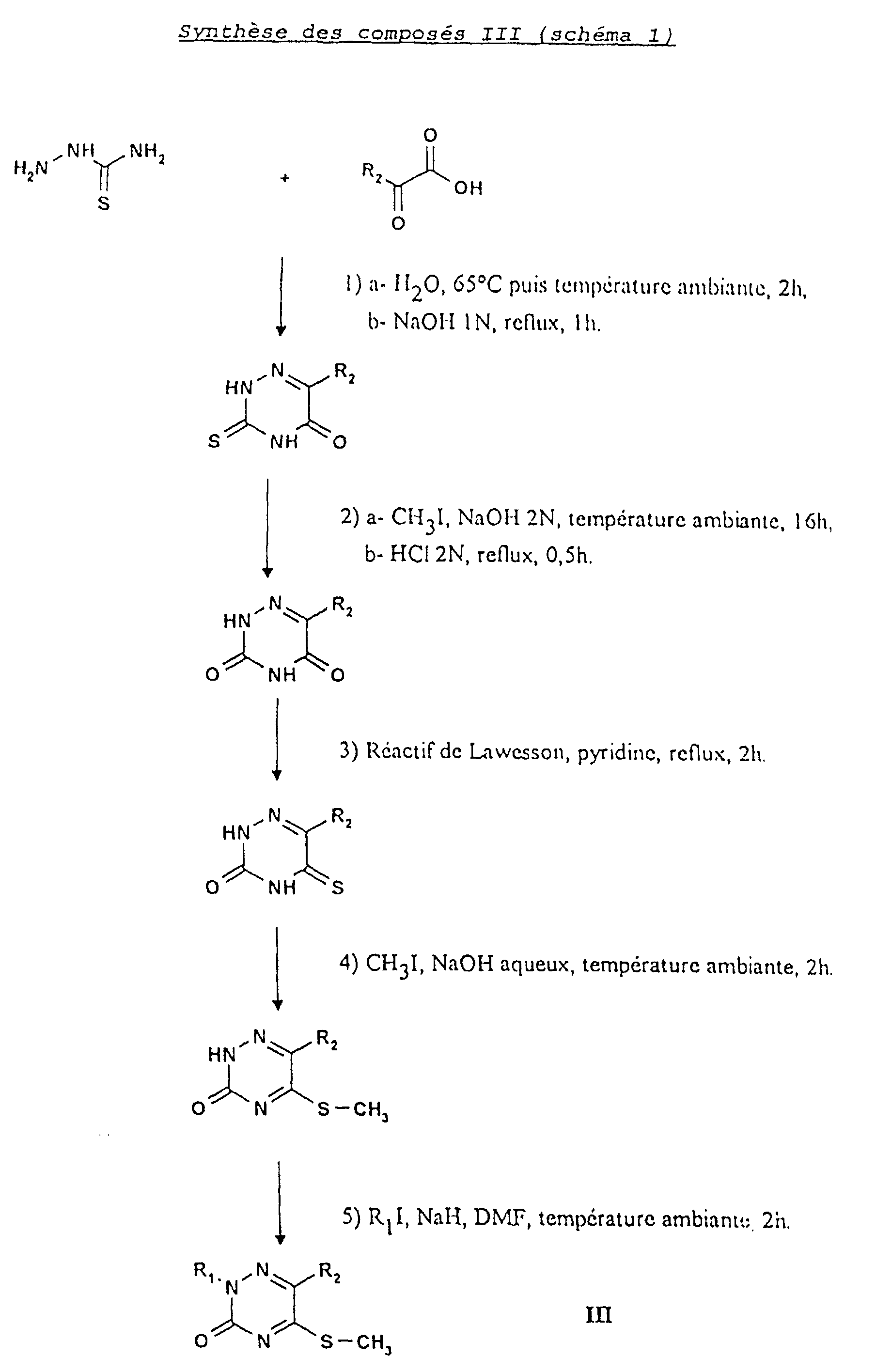

La condensation des intermédiaires III avec les alcools IV ou amines V est réalisée en présence d'une base telle que l'hydrure de sodium ou le tertbutylate de potassium dans le dioxane, le THF ou le toluène.

La séparation éventuelle des énantiomères ou diastéréoisomères de composés possédant un ou plusieurs carbones asymétriques est généralement réalisée sur les produits terminaux par chromatographie liquide sur colonne chirale.

### Synthèse des alcools B-OH IV

### A - Lorsque B représente un groupement de type

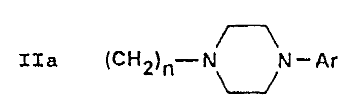

les alcools correspondant 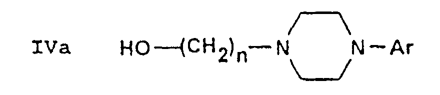 peuvent être obtenus
*1)* en traitant la pipérazine 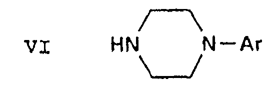 par un halogénoalcool

   VII HO―(CH₂)ₙ―Hal

   en présence de K₂CO₃ et éventuellement de KI, lorsque Hal = Br ou Cl, dans l'acétonitrile à reflux.
*2)* Lorsque n = 4 ou 5, en traitant la pipérazine VI par une lactone VIII 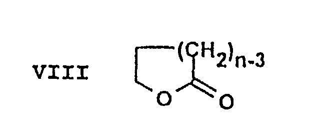 en présence d'un acide de Lewis tel que AlCl₃ et de triéthylamine dans le dichlorométhane, puis en réduisant l'amide IX formé 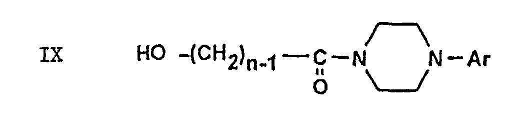 par un hydrure tel que AlLiH₄ dans le THF.
*3)* Lorsque Ar représente un hétérocycle de type pyrimidine en traitant la chloro-2 pyrimidine éventuellement substituée, par une hydroxyalcoylpipérazine X 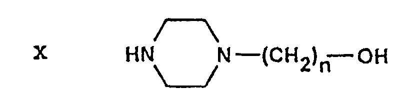 dans un solvant tel que, le toluène en présence d'une amine telle que la triéthylamine.

### B - Lorsque B représente un groupement de type

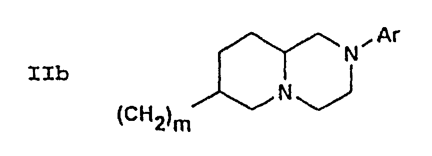

*1)* Lorsque m = 1
   Selon le procédé décrit dans le brevet PFIZER WO 93 25552 (1992).
*2)* Lorsque m = 2 en oxydant l'alcool XI 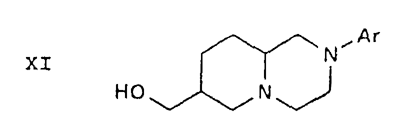 dans les conditions de Swern puis en traitant l'aldéhyde obtenu XII 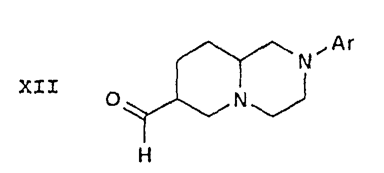 par un réactif de Wittig tel que le chlorure de méthoxyméthyltriphénylphosphonium dans le THF suivi de l'hydrolyse en milieu aqueux acide de l'éther d'énol obtenu XIII 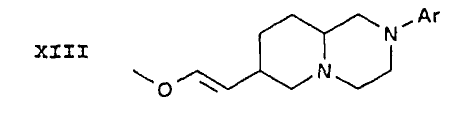 pour conduire à l'aldéhyde correspondant XIV 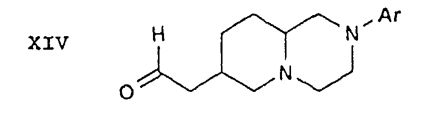 qui est réduit en alcool par un hydrure tel que NaBH₄ dans l'éthanol.

### C - Lorsque B représente un groupement de type

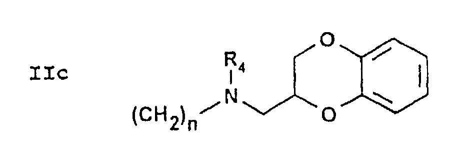

les alcools correspondants 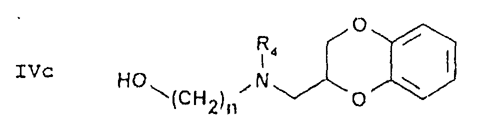 peuvent être préparés
*1)* en traitant la benzodioxaneméthylamine selon le procédé décrit au paragraphe A-2.
*2)* en condensant le benzodioxaneméthanol avec un aminoalcool XV

   XV HO―(CH₂)ₙ―NH₂

### Synthèse des amines B-NHR₃, V

### 1) Lorsque R₃ = H

Les composés V sont des amines commerciales ou peuvent être obtenus de façon classique telle que génération de l'amine primaire à partir du phtalimide intermédiaire.

### 2) Lorsque R₃ = Me

Les composés sont obtenus à partir des amines BNH₂ (dont la méthode de préparation est décrite ci-dessus) en formylant l'amine par l'anhydride formique dans un solvant tel que la pyridine pour conduire au formamide XVI 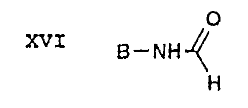 qui est réduit par un hydrure tel que LiAlH₄ dans le THF.

Les exemples suivants illustrent l'invention sans en limiter la portée.

Les analyses élémentaires et les spectres IR et RMN confirment les structures des composés obtenus selon l'invention.

### Exemple 1 : 2-Méthyl-5-[4-(4-pyrimidin-2-yl-pipérazin-1-yl)-butoxy]-2H-[1,2,4]-triazin-3-one (1).

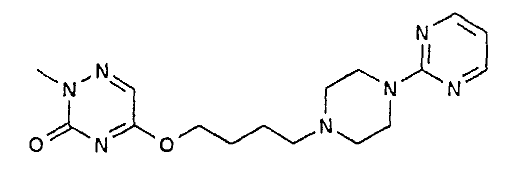

### a) 5-Thioxo-4,5 dihydro-2H-[1,2,4]triazin-3-one (1a).

Dans une solution de *2H*-[1,2,4]triazin-3,5 dione (50g; 442 mmol) dans 400 ml de pyridine sont ajoutés 98,3 g (243 mmol) de réactif de Lawesson. Le mélange est porté à reflux pendant 4 h. Après évaporation du solvant sous pression réduite, le résidu obtenu est repris par 400 ml d'eau. Le précipité brun qui se forme est isolé par filtration. Ces cristaux sont repris avec H₂O et extraits à l'acétate d'échyle. Après séchage (MgSO₄) et concentration à sec des phases organiques, on obtient des cristaux jaunes.

Le retraitement de la solution aqueuse (400 ml) par extraction à l'acétate d'éthyle permet d'isoler une nouvelle fraction de solide. Au total, après séchage, 60 g de cristaux jaunes sont obtenus.
F = 239°C
CCM gel de silice 60 F 254 Merck
CH₂Cl₂ - MeOH : 90-10 Rf = 0,4.

### b) 5-Méthyl sulfanyl-2H-[1,2,4]triazin-3-one (1b).

Le composé 1a (30 g; 232 mmol) et CH₃I (15,9 ml; 255 mmol) sont placés dans 300 ml d'eau. 18,6 g de NaOH (465 mmol) sont additionnés et le mélange est agité 1h à température ambiante. Le milieu réactionnel, refroidi sur lit de glace, est neutralisé à l'aide de 27 ml d'acide acétique puis extrait au dichlorométhane. Les phases organiques sont séchées (MgSO₄) puis concentrées à sec. Après recristallisation dans l'éther, on isole 29,9 g de composé 1b.
F = 171°C
CCM gel de silice 60 F 254 Merck
CH₂Cl₂ - MeOH : 90-10 Rf = 0, 5.

### c) 2-Méthyl-5-méthylsulfanyl-2H-[1,2,4]triazin-3-one (1c).

Une suspension de NaH (60 % dans l'huile de paraffine, 4,4 g; 110 mmol) dans 50 ml de DMF placée sous azote est refroidie à 0°C sur lit de glace. Le composé 1b (15,9 g; 111 mmol) dilué dans 100 ml de DMF est coulé goutte à goutte dans cette suspension. Le mélange est ensuite agité 1 h à température ambiante.

Après concentration à sec du milieu réactionnel, le résidu est repris par H₂O et extrait avec CH₂Cl₂. Les phases organiques sont séchées sur MgSO₄ puis évaporées sous pression réduite.

Après cristallisation dans EtOH/éther isopropylique, puis séchage, on isole 13,9 g de produit 1c sous forme de cristaux beiges.
F = 106°C
CCM gel de silice 60 F 254 Merck
CH₂Cl₂-MeOH : 95-5 Rf = 0,52.

### d) 4-hydroxy-1-(4-pyrimidin-2-yl-pipérazin-1-yl)-butan-1-one (1d).

AlCl₃ (18,5 g; 138,8 mmol) est placé sous atmosphère inerte (N₂) en suspension dans 100 ml de dichloroéthane. Sur ce mélange refroidi à 0°C sur lit de glace, une solution de triéthylamine (22,3 ml; 160 mmol) diluée dans 40 ml de dichloroéthane est coulée goutte à goutte.

Ce mélange est ramené à température ambiante puis une solution de 2-pyrimidinyl pipérazine (19,5 g; 119 mmol) et butyrolactone (8,2 ml; 107 mmol) dans 80 ml de dichloroéthane est ajoutée goutte à goutte.

Ce mélange est agité 2 h à température ambiante puis jeté sur 200 ml de glace. Après extraction au dichlorométhane, séchage (MgSO₄) et concentration à sec des phases organiques, on récupère 27 g de cristaux qui sont utilisés pour l'étape suivante sans purification supplémentaire.

### e) 4-(4-Pyrimidin-2-yl-pipérazin-1-yl)-butan-1-ol (1e).

LiAlH₄ (4,9 g; 129 mmol) est placé en suspension dans 100 ml de THF sous azote. 1d (27 g; 108 mmol) dilué dans 150 ml de THF est coulé goutte à goutte en veillant à maintenir la température à l'ambiante. Ce mélange est agité 1h, hydrolysé avec un minimum d'eau et séché sur MgSO₄. Après filtration des sels d'aluminium, le filtrat est concentré à sec. Le résidu obtenu est purifié par chromatographie flash sur silice (éluent : CH₂Cl₂ - MeOH : 95-5).

On récupère 17,3 g d'huile jaune. Après salification par l'acide fumarique dans l'éthanol, on isole un solide blanc.
F = 112°C
CCM gel de silice 60 F 254 Merck
CH₂Cl₂ - MeOH - NH₄OH : 90-9-1 Rf = 0,54.

### f) -2-Méthyl-5-[4-(4-pyrimidin-2-yl-pipérazin-1-yl)-butoxy]-2H-[1,2,4]-triazin-3-one (1).

NaH (60 % dans l'huile de paraffine; 0,68 g; 17 mmol) est placé sous atmosphère inerte dans le diméthoxyéthane (DME). A ce mélange refroidi à 0°C est ajoutée goutte à goutte une solution de composé 1e (sous forme de base; 4,5 g; 19 mmol) dilué dans 20 ml de DME.

Le milieu réactionnel est agité 1 h à température ambiante puis ramené à 0°C. Une solution de 1c (3 g; 19,1 mmol) dilué dans 40 ml de DME est alors coulée goutte à goutte. La température du milieu réactionnel est ensuite ramenée à l'ambiante et l'agitation est maintenue pendant 1 h.

Les minéraux sont filtrés sous vide et le filtrat concentré à sec. Le résidu obtenu est purifié par chromatographie flash sur silice (éluent CH₂Cl₂ - MeOH : 95-5) ce qui permet d'isoler une huile jaune qui cristallise.

Après recristallisation dans l'acétone, on obtient 2 g de composé 1.
F = 134°C
CCM gel de silice 60 F 254 Merck
CH₂Cl₂ - MeOH : 90-10 Rf = 0,46.

### Exemple 2 : 2-Méthyl-5-[4-[4-(4-méthyl-pyrimidin-2-yl)-pipérazin-1-yl]-butoxy]-2H-[1,2,4]triazin-3-one fumarate (2).

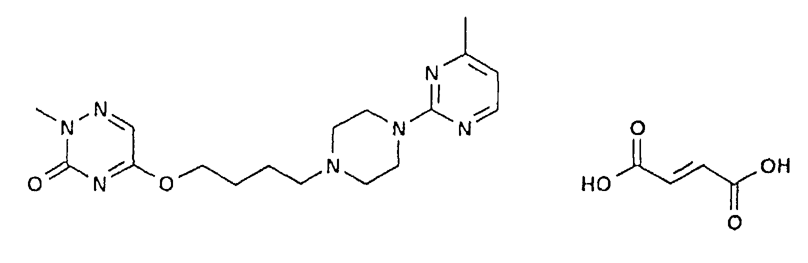

### a) 4-[4-(4-méthyl-pyrimidin-2-yl)-pipérazin-1-yl]-butan-1-ol (2a).

Le dichlorhydrate de 4-méthyl-2-pipérazin-1-yl-pyrimidine (7 g; 38,8 mmol) et le 4-chlorobutanol (4,6 ml; 46 mmol) sont placés dans 150 ml d'acétonitrile en présence de K₂CO₃ (21,5 g; 155,5 mmol). Ce mélange est porté à reflux pendant 16h. Après refroidissement, les minéraux sont éliminés par filtration et le filtrat est concentré à sec.

Le résidu obtenu est purifié par chromatographie - flash sur silice (éluent : CH₂Cl₂ - MeOH : 90-10). On obtient 4,7 g de cristaux blancs.
F = 76°C
CCM gel de silice 60 F 254 Merck
CH₂Cl₂ - MeOH : 90-10 Rf = 0,41.

### b) 2-Méthyl-5-[4-[4-(4-méthyl-pyrimidin-2-yl)-pipérazin-1-yl]-butoxy]-2H-[1,2,4]-triazin-3-one fumarate (2).

Ce composé est préparé selon le procédé décrit à l'étape f de l'exemple 1 en utilisant le 4-[4-(4-méthyl-pyrimidin-2-yl)-pipérazin-1-yl]-butan-1-ol, puis salifié par l'acide fumarique dans l'éthanol.
F = 144°C
CCM gel de silice 60 F 254 Merck
CH₂Cl₂ - MeOH - NH₄OH : 90-9-1 Rf = 0,7.

### Exemple 3 : 5-[4-[4-(4,6-Diméthyl-pyrimidin-2-yl)-pipérazin-1-yl]-butoxy]-2-mé-thyl-2H-[1,2,4]triazin-3-one fumarate (3).

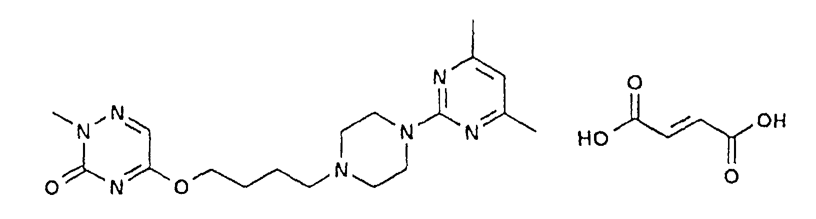

Ce composé est préparé selon le procédé décrit au stade f de l'exemple 1 en utilisant le 4-[4-(4,6-diméthyl-pyrimidin-2-yl)-pipérazin-1-yl]-butan-1-ol (préparé selon le procédé 2a) et le THF, puis salifié par l'acide fumarique dans l'éthanol.
F = 180°C
CCM gel de silice 60 F 254 Merck
CH₂Cl₂ - MeOH -NH₄OH : 90-9-1 Rf = 0,61.

### Exemple 4 : 5-[4-[4-(4-Méthoxy-pyrimidin-2-yl)-pipérazin-1-yl]-butoxy]-2-méthyl -2H-[1,2,4]triazin-3-one fumarate (4).

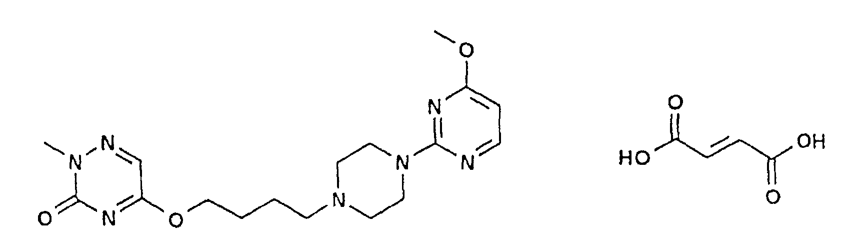

Ce composé est préparé selon le procédé décrit au stade f de l'exemple 1 en utilisant le 4-[4-(4-méthoxy-pyrimidin-2-yl)-pipérazin-1-yl]-butan-1-ol (préparé selon le procédé 2a) et le THF, puis salifié par l'acide fumarique dans l'éthanol.
F = 169°C
CCM gel de silice 60 F 254 Merck
CH₂Cl₂ - MeOH - NH₄OH : 90-9-1 Rf = 0,6.

### Exemple 5 : 5-[4-[4-(5-Méthoxy-pyrimidin-2-yl)-pipérazin-1-yl]-butoxy]-2-méthyl -2H-[1,2,4]triazin-3-one (5).

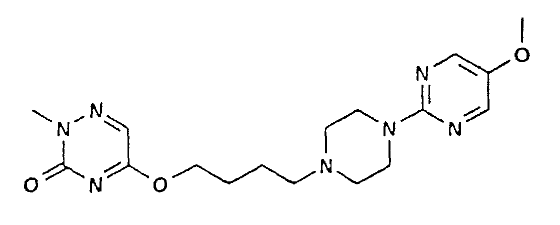

Ce composé est préparé selon le procédé décrit au stade f de l'exemple 1 en utilisant le 4-[4-(5-méthoxy-pyrimidin-2yl)-pipérazin-1-yl]-butan-1-ol (préparé selon le procédé 2a) et le dioxane.
F = 101°C
CCM gel de silice 60 F 254 Merck
CH₂Cl₂ - MeOH : 90-10 Rf = 0,58.

### Exemple 6 : 5-[4-[4-(4-Chloro-pyrimidin-2-yl)-pipérazin-1-yl]-butoxy]-2-méthyl-2H-[1,2,4]triazin-3-one (6).

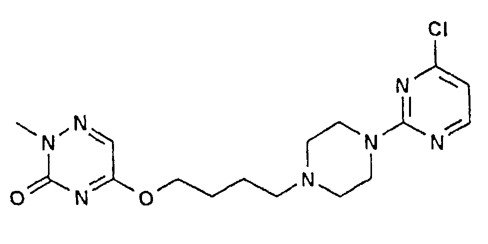

### a) 4-Pipérazin-1-yl-butan-1-ol (6a).

La pipérazine (50 g; 580 mmol) est placée dans 500 ml d'acétonitrile et le mélange est chauffé à 60°C. K₂CO₃ (96 g; 694 mmol) est additionné et le milieu réactionnel est porté à reflux. Le 4-chlorobutanol (58 ml; 581 mmol) est alors coulé goutte à goutte puis le reflux est maintenu pendant 4 h.

Après filtration des minéraux, le filtrat est concentré à sec sous vide. Le résidu obtenu est purifié par chromatographie flash sur silice (éluent : CH₂Cl₂ - MeOH - NH₄OH : 80-18-2) et on récupère 51 g d'huile claire 6a.
CCM gel de silice 60 F 254 Merck
CH₂Cl₂ - MeOH - NH₄OH : 80-18-2 Rf = 0,40.

### b) 4-[4-(4-chloro-pyrimidin-2-yl)-pipérazin-1-yl]-butan-1-ol (6b).

Le composé 6a (30 g; 189 mmol) et la triéthylamine (31,6 ml; 226 mmol) sont placés dans le toluène puis le mélange est porté à reflux. La 2,4 dichloro-pyrimidine (28,2 g; 189 mmol) est alors additionnée et le reflux maintenu pendant 3 h.

Après concentration à sec du milieu réactionnel, le résidu est repris par H₂O saturée par NaHCO₃ et extrait au dichlorométhane. Les phases organiques sont séchées sur MgSO₄ puis concentrées à sec. Le résidu obtenu est purifié par chromatographie flash sur silice (éluent CH₂Cl₂ - MeOH : 95-5) et on récupère 9,13 g de produit 6b sous forme d'huile.
CCM gel de silice 60 F 254 Merck
CH₂Cl₂ - MeOH : 95-5 Rf = 0,56.

### c) 5-[4-[4-(4-Chloro-pyrimidin-2-yl)-pipérazin-1-yl]-butoxy]-2-méthyl-2H-[1,2,4]-triazin-3-one (6).

Ce composé est préparé selon le procédé décrit au stade f de l'exemple 1 en utilisant le 4-[4-(4-chloro-pyrimidin-2-yl)-pipérazin-1-yl]-butan-1-ol 6b et le THF.
F = 96°C
CCM gel de silice 60 F 254 Merck
CH₂Cl₂ - MeOH - NH₄OH : 90-9-1 Rf = 0,6.

### Exemple 7 : 5-[4-[4-(5-Fluoro-pyrimidin-2-yl)-pipérazin-1-yl]-butoxy]-2-méthyl-2H-[1,2,4]triazin-3-one (7).

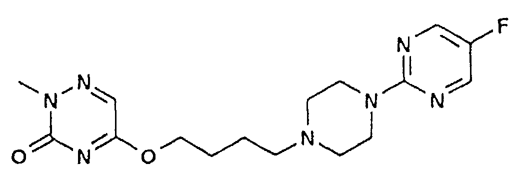

Ce composé est préparé selon le procédé décrit dans l'exemple 6 en utilisant à l'étape b la 5-fluoro-2-chloropyrimidine.
F = 102°C
CCM gel de silice 60 F 254 Merck
CH₂Cl₂ - MeOH : 90-10 Rf = 0,46.

### Exemple 8 : 2-Propyl-5-[4-(4-pyrimidin-2-yl-pipérazin-1-yl)-butoxy]-2H-[1,2,4]-triazin-3-one fumarate (8).

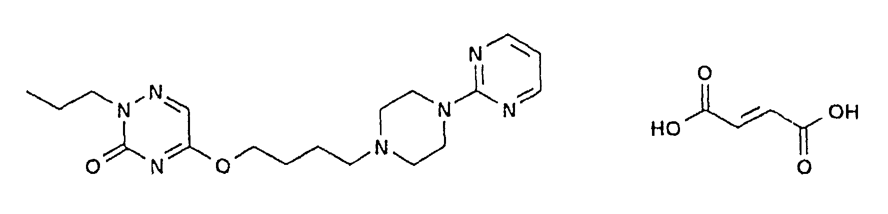

Ce composé est préparé selon le procédé décrit au stade f de l'exemple 1 en utilisant le 4-(4-pyrimidin-2-yl-pipérazin-1-yl)-butan-1-ol (préparé selon l'exemple 2a) et la 2-propyl-5-méthylsulfanyl-*2H*-[1,2,4]triazin-3-one (préparée selon l'exemple 1c en utilisant l'iodure de propyle) dans le THF, puis salifié par l'acide fumarique dans l'éthanol.
F = 131°C
CCM gel de silice 60 F 254 Merck
CH₂Cl₂ - MeOH - NH₄OH : 90-9-1 Rf = 0,55.

### Exemple 9 : 2,6-Diméthyl-5-[4-(4-pyrimidin-2-yl-pipérazin-1-yl)-butoxy]-2H-[1,2,4]-triazin-3-one (9).

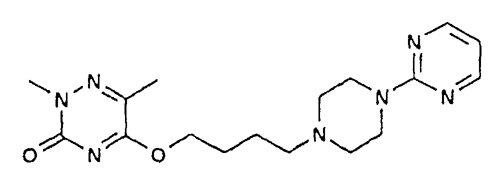

Ce composé est préparé selon le procédé décrit dans l'exemple 1 en utilisant la 6-méthyl-*2H*-[1,2,4]triazin-3,5 dione au stade a, le 4-(4-pyrimidin-2-yl-pipérazin-1-yl)-butan-1-ol (préparé selon le procédé 2a) et le dioxane au stade f.
F = 69°C
CCM gel de silice 60 F 254 Merck
CH₂Cl₂ - MeOH : 90-10 Rf = 0,47.

### Exemple 10 : 2-Méthyl-6-phényl-5-[4-(4-pyrimidin-2-yl-pipérazin-1-yl)-butoxy]-2H-[1,2,4]triazin-3-one (10).

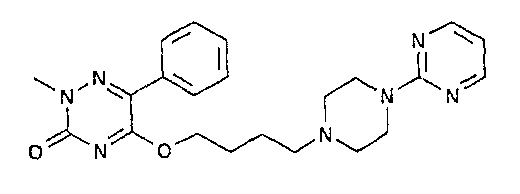

Ce composé est préparé selon le procédé décrit dans l'exemple 1 en utilisant la 6-phényl-*2H*-[1,2,4]triazin-3,5 dione au stade a, le 4-(4-pyrimidin-2yl-pipérazin-1-yl)-butan-1-ol (préparé selon le procédé 2a) et le dioxane au stade f.
F = 99°C
CCM gel de silice 60 F 254 Merck
CH₂Cl₂ - MeOH : 90-10 Rf = 0,48.

### Example 11 : 2-Méthyl-6-benzyl-5-[4-(4-pyrimidin-2-yl-pipérazin-1-yl)-butoxy]-2H-[1,2,4]triazin-3-one (11).

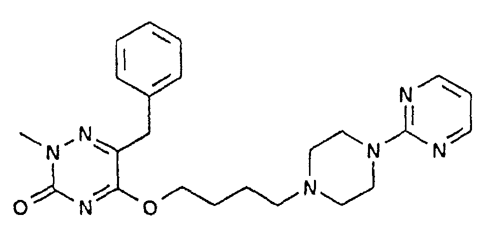

Ce composé est préparé selon le procédé décrit dans l'exemple 1 en utilisant la 6-benzyl-2*H*-[1,2,4]triazin-3,5 dione au stade a, le 4-(4-pyrimidin-2yl-pipérazin-1-yl)-butan-1-ol (préparé selon le procédé 2a) et le dioxane au stade f.
F = 86°C
CCM gel de silice 60 F 254 Merck
CH₂Cl₂ - MeOH : 90-10 Rf = 0,33.

### Exemple 12 : 2-Benzyl-5-[4-(4-pyrimidin-2-yl-pipérazin-1-yl)-butoxy]-2H-[1,2,4]-triazin-3-one (12).

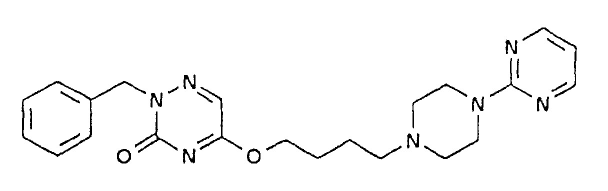

Ce composé est préparé selon le procédé décrit dans l'exemple 1 en utilisant le chlorure de benzyle au stade c, le 4-(4-pyrimidin-2-yl-pipérazin-1-yl)-butan-1-ol (préparé selon le procédé 2a) et le dioxane au stade f.
F = 86°C
CCM gel de silice 60 F 284 Merck
CH₂Cl₂ - MeOH : 90-10 Rf = 0,38

### Exemple 13 : 2-Méthyl-5-[3-(4-pyrimidin-2-yl-pipérazin-1-yl)-propoxy]-2H-[1,2,4]-triazin-3-one fumarate (13).

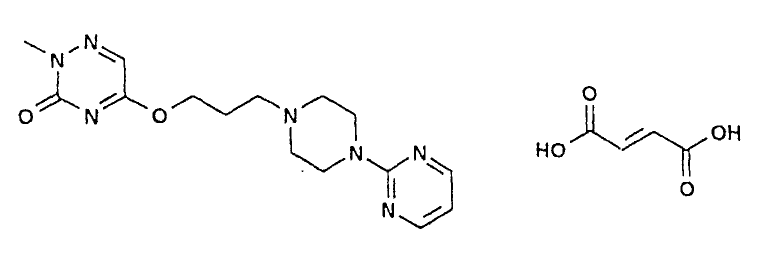

Ce composé est préparé selon le procédé décrit dans l'exemple 1 stade f en utilisant le 4-(4-pyrimidin-2-yl-pipérazin-1-yl)-propan-1-ol (préparé selon le procédé 2a à partir de 3-chloropropanol) et le dioxane, puis salifié par l'acide fumarique dans le méthanol.
F = 167°C
CCM gel de silice 60 F 254 Merck
CH₂Cl₂ - MeOH - NH₄OH : 90-9-1 Rf = 0,60

### Exemple 14 : 2-Méthyl-5-[5-(4-pyrimidin-2-yl-pipérazin-1-yl)-pentyloxy]-2H-[1,2,4]triazin-3-one (14).

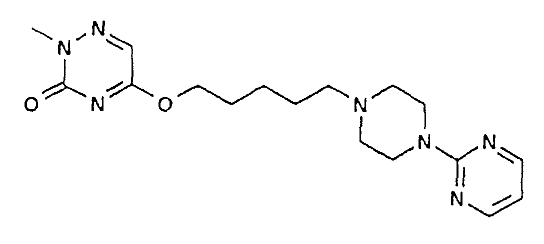

Ce composé est préparé selon le procédé décrit dans l'exemple 1 stade f en utilisant le 4-(4-pyrimidin-2-yl-pipérazin-1-yl)-pentan-1-ol (préparé selon le procédé 2a à partir du 5 choro-pentanol) et le dioxane.
F = 98°C
CCM gel de silice 60 F 254 Merck
CH₂Cl₂ - MeOH - NH₄OH : 90-9-1 Rf = 0,50

### Example 15 : 5-[4-[4-(3-Méthoxy-pyridin-2-yl)-pipérazin-1-yl]-butoxy]-2-méthyl-2H-[1,2,4]triazin-3-one fumarate (15).

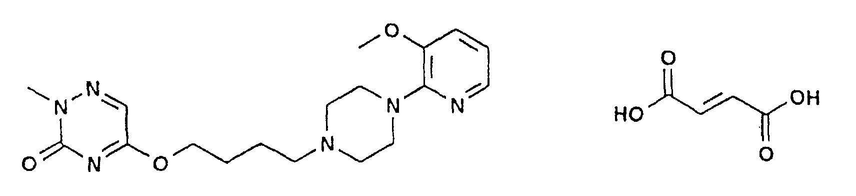

Ce composé est préparé selon le procédé décrit dans l'exemple 1 stade f en utilisant le 4-(4-(3-méthoxy-pyridin-2-yl)pipérazin-1-yl)-butan-1-ol (préparé selon l'exemple 2a) et le THF, puis salifié par l'acide fumarique dans l'éthanol.
F = 146°C
CCM gel de silice 60 F 254 Merck
CH₂Cl₂ - MeOH : 90-10 Rf = 0,38

### Exemple 16 : 5-[4-[4-(3-Chloro-phényl)-pipérazin-1-yl]-butoxy]-2-méthyl-2H-[1,2,4]triazin-3-one fumarate (16).

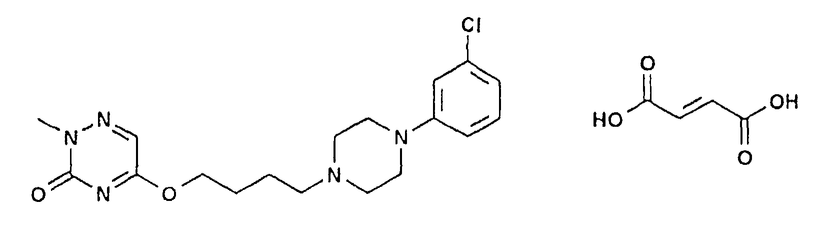

Ce composé est préparé selon le procédé décrit dans l'exemple 1 au stade f en utilisant le 4-(4-(3-chloro-phényl)-pipérazin-1-yl)-butan-1-ol (préparé selon l'exemple 2a) et le dioxane, puis salifié par l'acide fumarique dans l'éthanol.
F = 153°C
CCM gel de silice 60 F 254 Merck
CH₂Cl₂ - MeOH : 90-10 Rf = 0,47

### Exemple 17 : 5-[4-[4-(5-Chloro-2-méthoxy-phényl)-pipérazin-1-yl]-butoxy]-2-méthyl-2H-[1,2,4]triazin-3-one fumarate (17).

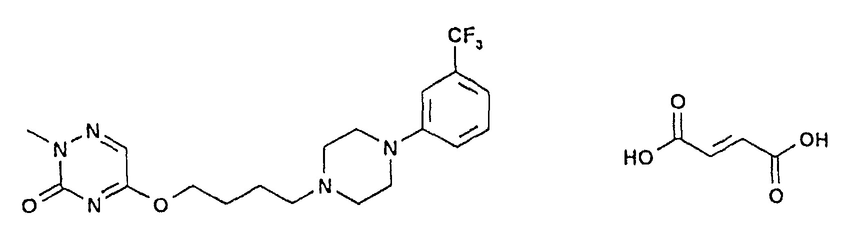

Ce composé est préparé selon le procédé décrit dans l'exemple 1 au stade f en utilisant le 4-(4-(3-trifluorométhyl-phényl)-pipérazin-1-yl)-butan-1-ol (préparé selon l'exemple 2a) et le dioxane, puis salifié par l'acide fumarique dans l'éthanol.
F = 184°C
CCM gel de silice 60 F 254 Merck
CH₂Cl₂ - MeOH : 90-10 Rf = 0,65

### Exemple 18 : 4-[4-[4-(2-Méthyl-3-oxo-2,3-dihydro-[1,2,4]triazin-5-yloxy)-butyl]-pipérazin-1-yl]-benzonitrile (18).

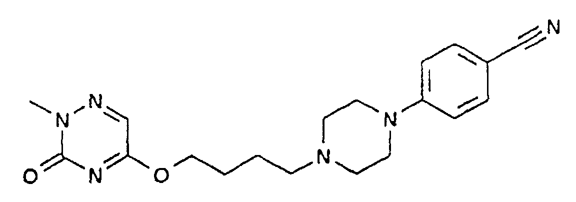

Ce composé est préparé selon le procédé décrit dans l'exemple 1 au stade f en utilisant le 4-(4-(4-hydroxy-butyl)-pipérazin-1-yl)-benzonitrile (préparé selon l'exem-ple 2a) et le dioxane.
F = 132°C
CCM gel de silice 60 F 254 Merck
CH₂Cl₂ - MeOH : 90-10 Rf = 0,56

### Exemple 19 : 5-[4-[4-(5-Chloro-2-méthoxy-phényl)-pipérazin-1-yl]-butoxy]-2-mé-thyl-2H-[1,2,4]triazin-3-one fumarate (19).

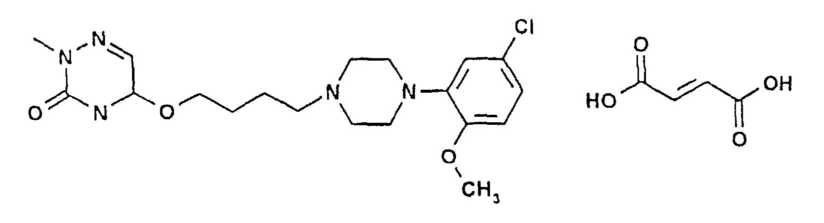

Ce composé est préparé selon le procédé décrit dans l'exemple 1 au stade f en utilisant le 4-(4-(5-chloro-2-méthoxyphényl)-pipérazin-1-yl)-butan-1-ol (préparé selon l'exemple 2a) et le dioxane, puis salifié par l'acide fumarique dans le méthanol.
F = 149°C
CCM gel de silice 60 F 254 Merck
CH₂Cl₂ - MeOH : 90-10 Rf *=* 0,54

### Exemple 20 : 5-[4-[(2,3-Dihydro-benzo[1,4]dioxin-2-ylméthyl)-amino]-butoxy]-2-méthyl-2H-[1,2,4]triazin-3-one fumarate (20).

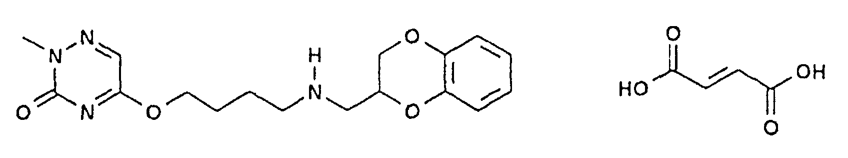

Ce composé est préparé selon l'exemple 1 en utilisant le C-(2,3-dihydro-benzo [1,4] dioxin-2-yl)-méthylamine au stade d, puis salifié par l'acide fumarique dans l'étha-nol.
F = 152°C
CCM gel de silice 60 F 254 Merck
CH₂Cl₂ - MeOH - NH₄OH : 90-9-1 Rf = 0,58

### Exemple 21 : (R) 5-[4-[(2,3-Dihydro-benzo[1,4]dioxin-2-ylméthyl)-amino]-buto-xy]-2-méthyl-2H-[1,2,4]triazin-3-one fumarate (21).

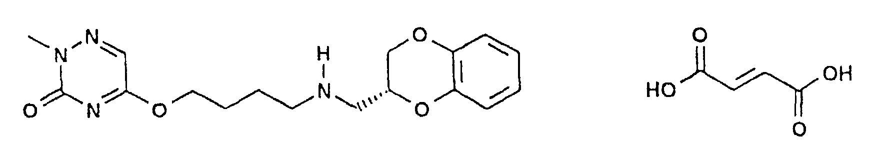

### a) (R) Trifluoro-méthanesulfonic acide -2,3-dihydrobenzo[1,4]dioxin-2-ylméthyl ester (21a).

Le (R) (2,3-dihydro-benzo-[1,4]dioxin-2-yl)-méthanol (3,6 g; 21,7 mmol) est placé dans 320 ml de dichlorométhane à - 5°C. L'anhydride triflique (3,3 ml; 19,7 mmol) dans 20 ml de CH₂Cl₂ est alors coulée goutte à goutte. Cc mélange est agité pendant 5 h à - 5°C.

Le milieu réactionnel est ensuite lavé par 50 ml d'HCl 1N puis par l'eau. La phase organique est séchée sur MgSO₄ puis concentrée à sec. L'huile 21a obtenue est utilisée sans autre purification dans l'étape suivante.

### b) (R) 4-[(2,3-Dihydro-benzo[1,4]dioxin-2-ylméthyl)-amino]-butan-1-ol (21b).

Le composé 21a est placé dans 30 ml de dichlorométhane. Le 4-aminobutanol (3,6 ml; 38,90 mmol) dilué dans 10 ml de CH₂Cl₂ est alors coulé goutte à goutte dans le milieu réactionnel. L'agitation du mélange est maintenue 14 h à température ambiante.

Le milieu réactionnel est concentré à sec et le résidu est repris par H₂O. Après extraction avec CH₂Cl₂, séchage des phases organiques sur MgSO₄ et concentration à sec, une huile est isolée. Elle est purifiée par chromatographie flash sur silice (éluent CH₂Cl₂ - MeOH - NH₄OH : 90-9-1) et on obtient 2,5 g d'huile claire 21b.
CCM gel de silice 60 F 254 Merck
CH₂Cl₂ - MeOH - NH₄OH : 90-9-1 Rf = 0,33

### c) (R) 5-[4-[(2,3-Dihydro-benzo[1,4]dioxin-2-ylméthyl)-amino]-butoxy]-2-méthyl-2H-[1,2,4]triazin-3-one fumarate (21).

Le composé final est obtenu selon le procédé décrit dans l'exemple 1 stade f en utilisant l'amino alcool 21b et le dioxane, puis salifié par l'acide fumarique dans le méthanol.
F = 137°C
CCM gel de silice 60 F 254 Merck
CH₂Cl₂ - MeOH - NH₄OH : 90-9-1 RF= 0,65

### Exemple 22 : (S) 5-[4-[(2,3-Dihydro-benzo[1,4]dioxin-2-ylméthyl)-amino]-buto-xy]-2-méthyl-2H-[1,2,4]triazin-3-one fumarate (22).

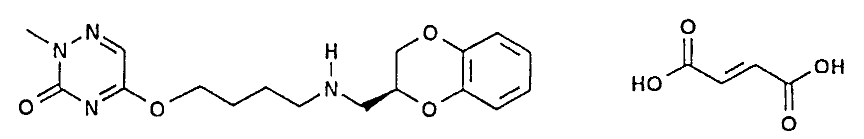

Ce composé est obtenu selon le procédé décrit dans l'exemple 21 en utilisant le (S) (2,3-dihydro-benzo-[1,4]dioxin-2-yl)-méthanol.
F = 135°C
CCM gel de silice 60 F 254 Merck
CH₂Cl₂ - MeOH - NH₄OH : 90-9-1 Rf = 0,59

### Exemple 23 : Trans 2-méthyl-5-(2-pyrimidin-2-yl-octahydropyrido[1,2-a]pyra-zin-7-yl-méthoxy)-2H-[1,2,4]triazin-3-one (23).

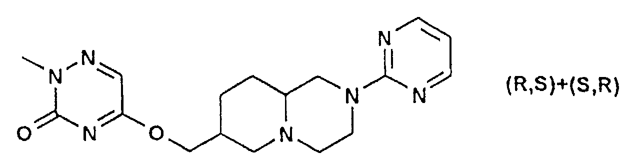

Ce composé est obtenu selon le procédé décrit dans l'exemple 1 au stade f en utilisant le trans (2-pyrimidin-2-yl-octahydro-pyrido-[1,2a]pyrazin-7-yl)-méthanol et le tertbutylate de potassium.
F = 140°C
CCM gel de silice 60 F 254 Merck
CH₂Cl₂ - MeOH : 90-10 Rf = 0,35

### Exemple 24 : Trans 2-méthyl-5-[2-(2-pyrimidin-2-yl-octahydropyrido[1,2-a]py-razin-7-yl)-éthoxy]-2H-[1,2,4]triazin-3-one (24).

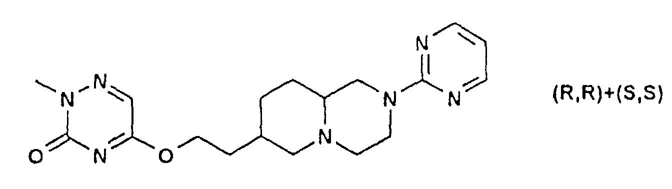

### a) 2-Pyrimidin-2-yl-octahydro-pyrido[1,2-a]pyrazine-7-carbaldehyde (24a).

Le chlorure d'oxalyle (6,2 ml; 68,9 mmol) est placé dans 220 ml de dichlorométhane à - 50°C. Le DMSO (10 ml; 140,9 mmol) dilué dans 25 ml de dichlorométhane est ajouté. Sur ce mélange maintenu à - 50°C, le trans (2-pyrimidin-2-yl-octahydro-pyrido-[1,2a]-pyrazin-7-yl]-méthanol dilué dans 30 ml de CH₂Cl₂ est coulé goutte à goutte.

Après 0,5 h d'agitation à - 50°C, la triéthylamine (40,8 ml; 293 mmol) est ajoutée et la température du milieu réactionnel est ramenée à l'ambiante.

Le milieu réactionnel est lavé avec H₂O puis la phase organique séchée sur MgSO₄ puis concentré à sec. L'huile obtenue 24a est purifiée par chromatographie flash CH₂Cl₂ - MeOH : 90-10.
CCM gel de silice 60 F 254 Merck
CH₂Cl₂ - MeOH - NH₄OH : 90-9-1 Rf = 0,24

### b) (2-Pyrimidin-2-yl-octahydro-pyrido[1,2-a]pyrazin-7-yl)-acétaldéhyde (24b).

Le chlorure de méthoxyméthyltriphénylphosphonium (41,4 g; 120,8 mmol) et la diisopropylamine (11,6 ml; 88,5 mmol) sont placés dans 180 ml de THF à 0°C sous atmosphère inerte. Le n-butyllithium (solution 1,6 M dans le THF; 55,2 ml; 88,3 mmol) est coulé goutte à goutte et ce mélange est agité 1 h à température ambiante. Le milieu réactionnel est ramené à 0°C et 24a dilué dans 120 ml de THF est coulé goutte à goutte. Ce mélange est agité 1 nuit à température ambiante puis concentré à sec.

Le résidu est repris par H₂O et extrait à l'acétate d'éthyle. Les phases organiques sont lavées avec H₂O acide (pH = 1). Cette phase aqueuse est lavée par AcOEt puis ramenée à pH 12 (NaOH concentrée) et extraite au dichlorométhane. Ces phases organiques sont séchées (MgSO₄) puis concentrées à sec. L'huile obtenue est purifiée deux fois par chromatographie flash sur silice (éluent CH₂Cl₂ - MeOH : 90 - 10 et AcOEt - acétone : 50 - 50). On récupère 6,3 g d'huile jaune.
CCM gel de silice 60 F 254 Merck
CH₂Cl₂ - MeOH - NH₄OH : 90-9-1 Rf = 0,34

### c) 2-(2-Pyrimidin-2-yl-octahydro-pyrido[1,2-a]pyrazin-7-yl)-éthanol (24c).

NaBH₄ (1 g; 10,2 mmol) est placé dans 30 ml d'éthanol puis 24b (6,3 g; 24,2 mmol) dilué dans 40 ml d'éthanol est coulé goutte à goutte. Ce mélange est agité une nuit à température ambiante, puis hydrolysé avec de l'eau.

Après extraction au dichlorométhane, les phases organiques sont séchées (MgSO₄) puis concentrées à sec. Le résidu obtenu est purifié par chromatographie flash sur silice (éluent CH₂Cl₂ - MeOH : 90 - 10). On récupère 4,5 g d'huile jaune.
CCM gel de silice 60 F 254 Merck
CH₂Cl₂ - MeOH : 90-10 Rf = 0,12

### d) Trans 2-méthyl-5-[2-(2-pyrimidin-2-yl-octahydropyrido[1,2-a]pyrazin-7-yl)-éthoxy]-2H-[1,2,4]triazin-3-one (24).

Ce composé est obtenu selon le procédé décrit dans l'exemple 1 au stade f en utilisant l'alcool 24c et le dioxane.
F : 145°C
CCM gel de silice 60 F 254 Merck
CH₂Cl₂ - MeOH : 90-10 Rf = 0,33

### Exemple 25 : Trans (-) 2-méthyl-5-[2-(2-pyrimidin-2-yl-octahydro-pyrido[1,2-a]-pyrazin-7-yl)-éthoxy]-2H-[1,2,4]triazin-3-one (25).

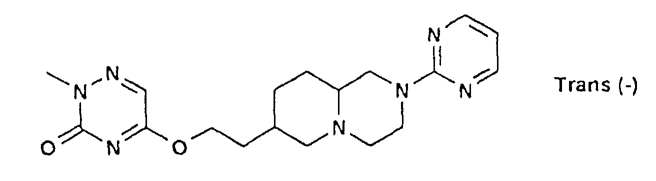

Le racémique préparé selon l'exemple 24 est séparé par chromatographie HPLC préparative [silice greffée Chiracel OD-20 µ, éluent : hexane - isopropanol : 65 - 35 et 1/1000 de diéthylamine). Une huile est isolée et purifiée à nouveau par chromatographie flash (éluent CH₂Cl₂ - MeOH : 90-10). Après cristallisation dans le mélange éther/éther isopropylique, on isole 0,31 g de cristaux blancs.
F = 145°C
CCM gel de silice 60 F 254 Merck
CH₂Cl₂ - MeOH : 90-10 Rf = 0,36

### Exemple 26 : Trans (+) 2-méthyl-5-[2-(2-pyrimidin-2-yl-octahydro-pyrido[1,2-a]-pyrazin-7-yl)-éthoxy]-2H-[1,2,4]triazin-3-one (26).

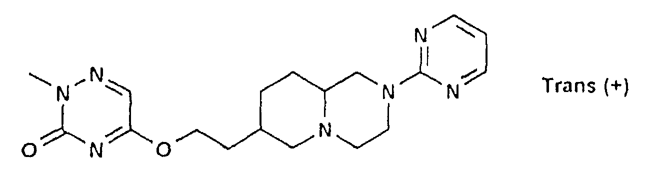

Ce composé est isolé selon le procédé décrit dans l'exemple 25. On isole 0,42 g de solide blanc.
F = 146°C
CCM gel de silice 60 F 254 Merck
CH₂Cl₂ - MeOH : 90-10 Rf = 0,36

### Exemple 27 : 2-Méthyl-5-[4-(4-pyrimidin-2-yl-pipérazin-1-yl)-butylamino]-2H-[1,2,4]triazin-3-one (27).

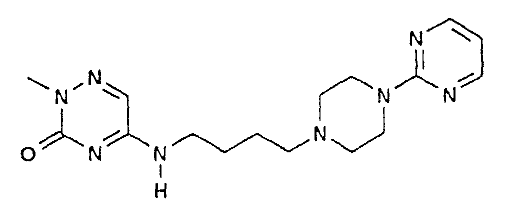

### a) 4-(4-Pyrimidin-2-yl-pipérazin-1-yl)-butylamine (27a).

Le dichlorhydrate de pyrimidine-2-yl-pipérazine (17,5 g; 73,8 mmol) et le 4-bromobutylphtalimide (25 g; 88 mmol) sont placés dans 200 ml de n-butanol et chauffés à reflux pendant 8 h. Après concentration à sec du milieu réactionnel, le résidu obtenu est repris par 100 ml d'éthylenediamine et chauffé à reflux pendant 5h.

Le milieu réactionnel est concentré sous vide, le résidu repris par H₂O basique (pH = 11) et cette phase aqueuse est extraite au dichlorométhane. Les phases organiques sont séchées sur MgSO₄ puis concentrées à sec. Le résidu obtenu est purifié par chromatographie flash sur silice (CH₂Cl₂ - MeOH - NH₄OH : 90-9-1) et on récupère 10 g d'huile correspondant au produit 27a.
CCM gel de silice 60 F 254 Merck
CH₂Cl₂ - MeOH - NH₄OH : 80-18-2 Rf = 0,24

### b) 2-Méthyl-5-[4-(4-pyrimidin-2-yl-pipérazin-1-yl]-butylamino]-2H-[1,2,4]triazin-3-one (27).

L'amine 27a (2,2 g; 9,3 mmol) est placée en présence de 1c (1,7 g; 11,2 mmol) dans 30 ml de toluène et le mélange est chauffé à reflux pendant 4h. Le milieu réactionnel est concentré à sec sous vide, le résidu est repris par H₂O/NaHCO₃ et extrait par le dichlorométhane. Les phases aqueuses sont séchées sur MgSO₄ puis concentrées à sec. Le résidu obtenu est purifié par chromatographie flash sur silice (éluent CH₂Cl₂ - MeOH : 90-10) et on obtient 2,50 g de cristaux blancs.
F = 188°C
CCM gel de silice 60 F 254 Merck
CH₂Cl₂ - MeOH : 90-10 Rf : 0,26

Les composés de l'invention ont été soumis à des essais pharmacologiques qui ont mis en évidence leur intérêt comme substances actives en thérapeutique.

### Liaison aux récepteurs 5-HT_{1A}, D₂ dopaminergiques et α1-adrénergiques :

Des cerveaux de rats mâles Sprague Dawley 180-200 g [Ico : OFA SD (I.O.P.S. Caw) ; Iffa Credo, France], maintenus à - 70°C ont été utilisés dans toutes les études.

L'affinité des produits pour les différents récepteurs a été déterminée par déplacement de ligands radioactifs dans les conditions résumées dans le tableau 1.

La réaction est stoppée par filtration rapide, sous vide, sur filtres Whatman GF/B, les tubes sont rincés avec 2 x 5 ml de tampon Tris-HCl 50 mM, pH 7,4 à 25°C. La radioactivité recueillie sur le filtre est analysée en scintillation liquide après ajout de 4 ml de liquide scintillant (Emulsifier Safe, Packard). Toutes les expériences sont réalisées en triple.

Les constantes d'inhibition (Ki) des produits sont estimées à partir des expérimentations de déplacement en utilisant le programme de régression non-linéaire RADLIG version 4 de EBDA (equilibrium binding data analysis) (Biosoft, Cambridge, UK; McPherson, 1985).

Les valeurs de pKi (-log Ki) sont données sous forme de moyenne ± SEM d'au moins 3 expérimentations (tableau 2).

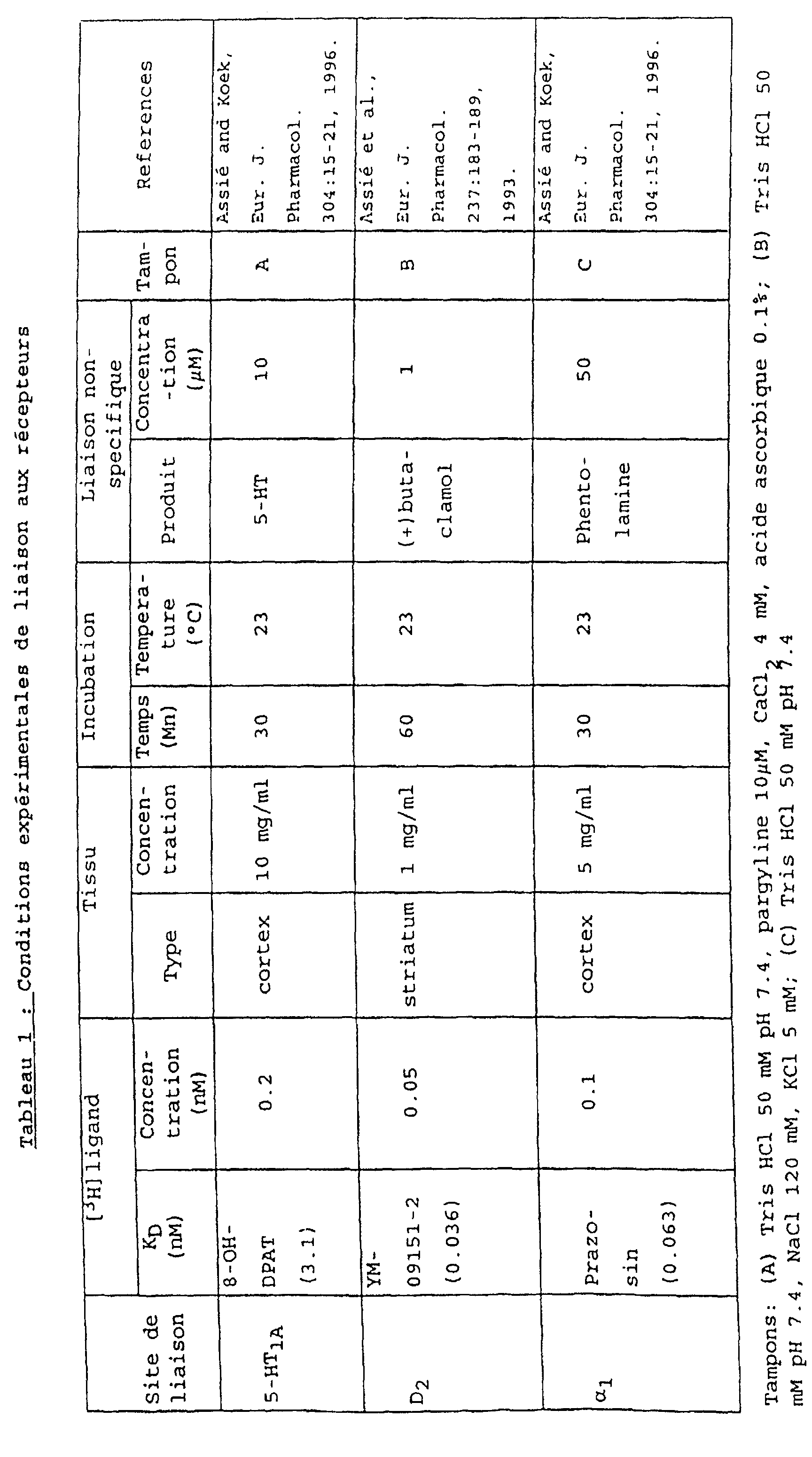

**Tableau 2**

| Composé n° | pKi | | |
|---|---|---|---|
| | 5HT_{1A} | α₁ | D₂ |
| 1 | 9,17 | 6,25 | 5,80 |
| | | | |
| 2 | 9,57 | 6,11 | 5,76 |
| | | | |
| 3 | 9,56 | 6,01 | 5,95 |
| | | | |
| 4 | 9,86 | 6,74 | 5,65 |
| | | | |
| 6 | 9,68 | 6,25 | 5,77 |
| | | | |
| 7 | 9,04 | | |
| | | | |
| 15 | 9,58 | | |
| | | | |
| 16 | 10,12 | | |
| | | | |
| 17 | 9,89 | | |
| | | | |
| 19 | 9,51 | 8,21 | 7,82 |
| | | | |
| 20 | 9,97 | 7,65 | 7,18 |
| | | | |
| 21 | 9,48 | | |
| | | | |
| 22 | 10,47 | | |
| | | | |
| 24 | 9,28 | 6,35 | 5,54 |
| | | | |
| 26 | 9,61 | | |
| | | | |
| Buspirone | 7,65 | 6,19 | 7,49 |
| | | | |
| Flesinoxan | 8,91 | 6,50 | 7,05 |

### Syndrome sérotoninergique :

L'activité centrale des composés de l'invention a été évaluée par leur capacité à provoquer le syndrome 5-HT qui est caractérisé par :
- une flexion et une extension alternée des pattes avant (reciprocal fore-paw treading: FPT)
- la rétraction de la lèvre inférieure (lower lip retraction : LLR)
- une position ou la surface ventrale de l'animal est en contact avec le sol et les pattes arrières étendues (flat body posture : FBP).

Les expériences de l'évaluation du syndrome 5-HT sont réalisées chez le rat mâle (Sprague-Dawley) selon la technique décrite par F.C. COLPAERT et al. (Drug. Dev. Res., 26, 21-48; 1992) et M.S. KLEVEN et al. (J.P.E.T., 282 : 747-759, 1997).

Le tableau 3 donne, à titre d'exemple, les doses actives (ED₅₀) pour certains dérivés de l'invention par rapport aux produits de référence tels que Buspirone et Flesinoxan.

**Tableau 3**

| Syndrome 5-HT | | | |
|---|---|---|---|
| Composé n° | ED₅₀ mg/kg po | | |
| | FBP | LLR | FPT |
| 1 | 0,08 | 0,02 | 0,08 |
| | | | |
| 2 | 0,08 | 0,02 | 0,08 |
| | | | |
| 3 | 0,08 | 0,08 | 0,08 |
| | | | |
| 4 | 0,08 | <0,04 | 0,08 |
| | | | |
| 6 | 0,08 | 0,08 | 0,08 |
| | | | |
| 7 | 0,31 | 0,08 | 0,31 |
| | | | |
| 17 | 0,31 | 0,08 | 0,31 |
| | | | |
| 20 | 0,08 | <0,04 | 0,31 |
| | | | |
| 24 | 0,08 | <0,04 | 0,08 |
| | | | |
| 26 | <0,04 | <0,04 | 0,04 |
| | | | |
| Buspirone | 20 | 2,5 | >40 |
| | | | |
| Flesinoxan | 1,25 | 1,25 | 5 |

### Activité antidépressive : Test de la nage forcée :

Les composés de l'invention sont testés selon la procédure décrite par R. PORSOLT et al. (Eur. J. Pharmacol. 47 : 379-391 ; 1978).

Les doses actives (ED₅₀) sont calculées pour chaque composé en fonction du pourcentage d'animaux présentant une diminution significative par rapport aux animaux témoins (p < 0.05) du temps d'immobilité (tableau 4).

**Tableau 4**

| Composé n° | ED₅₀ mg/kg po |
|---|---|
| 2 | 0,04 |
| | |
| 6 | 0,63 |
| | |
| 24 | 0,04 |
| | |
| 26 | 0,04 |
| | |
| Buspirone | >160 |
| | |
| Flesinoxan | 1,25 |

Les résultats des différents essais montrent que les composés de formule générale I possèdent in vitro, une haute affinité pour les récepteurs sérotoninergiques de type 5HT_{1A} et une bonne sélectivité vis-à-vis des récepteurs α₁ et D₂. In vivo, ils montrent une activité agoniste vis-à-vis des récepteurs 5HT_{1A} et sont puissamment actifs sur des modèles comportementaux tel que le test de la nage forcée.

Les composés de l'invention peuvent donc être utiles dans le traitement de l'anxiété, la dépression, la douleur, la neurodégénérescence, la schizophrénie, la maladie d'Alzheimer, les troubles du sommeil, pour la régularisation de prise de nourriture, pour la régularisation de la sécrétion gastrique, et pour le traitement des désordres vasculaires, cardiovasculaires et cérébro-vasculaires tels que l'hypertension ou la migraine.

Les préparations pharmaceutiques contenant comme principe actif des composés de formule générale I peuvent être mises en forme pour l'administration par voie orale, rectale ou parentérale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

## Revendications

1. Dérivés de 3-oxo-(2H)-1,2,4-triazine correspondant à la formule générale **I** 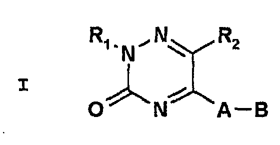 dans laquelle
- **R**_{**1**} représente
• l'hydrogène, uniquement lorsque A est un atome d'azote éventuellement substitué
• un groupe alcoyle en C₁-C₄ linéaire ou ramifié
• un groupe phénylalcoyle en C₁-C₄, le noyau phényle étant éventuellement substitué par un ou plusieurs groupements alcoyle en C₁-C₄, alcoxy en C₁-C₃, halogène ou trifluorométhyle.
- **R**_{**2**} représente
• l'hydrogène
• un radical alcoyle en C₁-C₄ linéaire ou ramifié
• un groupement phényle ou phénylalcoyle en C₁-C₄, le noyau phényle étant éventuellement substitué par un ou plusieurs groupements alcoyle en C₁-C₄, alcoxy en C₁-C₃, halogène ou trifluorométhyle.
- **A** représente un atome d'oxygène ou un atome d'azote éventuellement substitué NR₃.
- **R**_{**3**} représente l'hydrogène ou un groupe méthyle.
- **B** représente un groupement de type
• 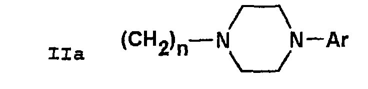 dans lequel **Ar** représente lui-même une structure aromatique phényle, pyridyle ou pyrimidyle, éventuellement substituée par un ou plusieurs groupements alcoyle en C₁-C₃, alcoxy en C₁-C₃, hydroxy, trifluorométhyle ou halogène et n peut prendre les valeurs entières de 3 à 5.
• 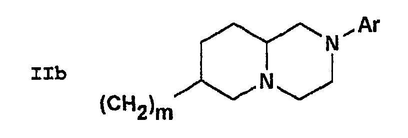 dans lequel Ar est tel que défini dans la formule **IIa** et **m** peut prendre les valeurs entières 1 et 2
• 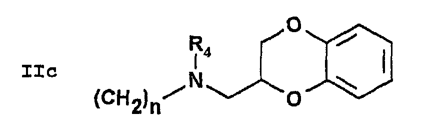 dans lequel **R**_{**4**} représente l'hydrogène ou un groupement alcoyle en C₁-C₃ et **n** peut prendre les valeurs entières de 3 à 5.
Les composés de formule générale **I** se présentant sous la forme aussi bien de mélanges racémiques que de différents énantiomères ou diastéréoisomères purs ou de leurs mélanges,
ainsi que les sels minéraux ou organiques thérapeutiquement acceptables des composés de formule générale **I**.

2. Composé selon la revendication **1** **caractérisé en ce qu'**il est choisi parmi les composés suivants :
* 2-Méthyl-5-[4-(4-pyrimidin-2-yl-piperazin-1-yl)-butoxy]-*2H*-[1,2,4]-triazin-3-one.
* 2-Méthyl-5-[4-[4-(4-méthyl-pyrimidin-2-yl)-pipérazin-1-yl]-butoxy]-*2H*-[1,2,4]tria-zin-3-one fumarate.
* 5-[4-[4-(4,6-Diméthyl-pyrimidin-2-yl)-pipérazin-1-yl]-butoxy]-2-méthyl-*2H-*[1,2,4]-triazin-3-one fumarate.
* 5-[4-[4-(4-Méthoxy-pyrimidin-2-yl)-pipérazin-1-yl]-butoxy]-2-méthyl-*2H*-[1,2,4]-triazin-3-one fumarate.
* 5-[4-[4-(5-Méthoxy-pyrimidin-2-yl)-pipérazin-1-yl]-butoxy]-2-méthyl-*2H*-[1,2,4]-triazin-3-one.
* 5-[4-[4-(4-Chloro-pyrimidin-2-yl)-pipérazin-1-yl]-butoxy]-2-méthyl-*2H*-[1,2,4]-tria-zin-3-one.
* 5-[4-[4-(5-Fluoro-pyrimidin-2-yl)-pipérazin-1-yl]-butoxy]-2-méthyl-*2H*-[1,2,4]-tria-zin-3-one.
* 2-Propyl-5-[4-(4-pyrimidin-2-yl-pipérazin-1-yl)-butoxy]-*2H*-[1,2,4]triazin-3-one fumarate.
* 2,6-Diméthyl-5-[4-[4-pyrimidin-2-yl-pipérazin-1-yl)-butoxy]-*2H*-[1,2,4] triazin-3-one.
* 2-Méthyl-6-phényl-5-[4-(4-pyrimidin-2-yl-pipérazin-1-yl)-butoxy]-*2H*-[1,2,4]-tria-zin-3-one.
* 2-Méthyl-6-benzyl-5-[4-(4-pyrimidin-2-yl-pipérazin-1-yl)-butoxy]-*2H*-[1,2,4]-tria-zin-3-one.
* 2-Benzyl-5-[4-(4-pyrimidin-2-yl-pipérazin-1-yl)-butoxy]*-2H*-[1,2,4]-triazin-3-one.
* 2-Méthyl-5-[3-(4-pyrimidin-2-yl-pipérazin-1-yl]-propoxy]*-2H*-[1,2,4]-triazin-3-one fumarate.
* 2-Méthyl-5-[5-(4-pyrimidin-2-yl-pipérazin-1-yl)-pentyloxy]*-2H*-[1,2,4]-triazin-3-one.
* 5-[4-[4-(3-Méthoxy-pyridin-2-yl)-pipérazin-1-yl]-butoxy]-2-méthyl-*2H*-[1,2,4]-triazin-3-one fumarate.
* 5-[4-[4-(3-Chloro-phényl)-pipérazin-1-yl]-butoxy]-2-méthyl-*2H*-[1,2,4]triazin-3-one fumarate.
* 5-[4-[4-(5-Chloro-2-méthoxy-phényl)-pipérazin-1-yl]-butoxy]-2-méthyl-*2H*- [1,2,4]-triazin-3-one fumarate.
* 4-[4-[4-(2-Méthyl-3-oxo-2,3-dihydro-[1,2,4]triazin-5-yloxy)-butyl]-pipérazin-1-yl]-benzonitrile.
* 5-[4-[4-(5-Chloro-2-méthoxy-phényl)-pipérazin-1-yl]-butoxy]-2-méthyl-*2H*-[1,2,4]-triazin-3-one fumarate.
* 5-[4-[(2,3-Dihydro-benzo[1,4]dioxin-2-ylméthyl)-amino]-butoxy]-2-méthyl-*2H*-[1,2,4]-triazin-3-one fumarate.
* (R) 5-[4-[(2,3-Dihydro-benzo[1,4]dioxin-2-ylméthyl)-amino]-butoxy]-2-méthyl-*2H*-[1,2,4]-triazin-3-one fumarate.
* (S) 5-[4-[(2,3-Dihydro-benzo[1,4]dioxin-2-ylméthyl)-amino]-butoxy]-2-méthyl-*2H*-[1,2,4]-triazin-3-one fumarate.
* Trans 2-méthyl-5-(2-pyrimidin-2-yl-octahydro-pyrido[1,2-a]pyrazin-7-yl-méthoxy)-*2H*-[1,2,4]triazin-3-one.
* Trans (-) 2-méthyl-5-[2-(2-pyrimidin-2-yl-octahydro-pyrido[1,2-a]pyrazin-7-yl)-éthoxy]*-2H-*[1,2,4]triazin-3-one.
* Trans (+) 2-méthyl-5-[2-(2-pyrimidin-2-yl-octahydro-pyrido[1,2-a]pyrazin-7-yl-éthoxy]-*2H*-[1,2,4]triazin-3-one.
• 2-Méthyl-5-[4-(4-pyrimidin-2-yl-pipérazin-1-yl)-butylamino]-*2H*-[1,2,4]triazin-3-one.

3. Procédé de préparation des composés chimiques selon les revendications **1** et **2 caractérisé :**
**en ce que** l'on traite un dérivé de formule générale **III** 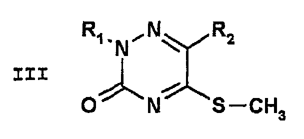 avec un alcool B-OH **IV** ou une amine BNHR₃ V, R₁, R₂, R₃ et B étant tels que définis dans la revendication **1**, en présence d'hydrure de sodium ou de tertiobutylate de potassium, dans le dioxane, le tétrahydrofurane ou le toluène.

4. A titre de médicaments utilisables dans le traitement des maladies nécessitant des agonistes de récepteurs 5HT_{1A}, les composés définis selon l'une des revendications **1** et **2**.

5. A titre de médicaments utiles, par exemple, pour le traitement de l'anxiété, la dépression, la douleur, la neurodégénérescence, la schizophrénie, la maladie d'Alzheimer, les troubles du sommeil, la régularisation de prise de nourriture, la régularisation de la sécrétion gastrique, le traitement des désordres vasculaires, cardiovasculaires et cérébrovasculaires, les composés définis selon l'une des revendications **1** et **2**.

6. Composition pharmaceutique **caractérisée en ce qu'**elle contient à titre de principe actif un composé défini selon l'une des revendications **1** et **2.**

7. Composition pharmaceutique selon la revendication 6, **caractérisée en ce qu'**elle contient un composé défini selon l'une des revendications **1** et **2** en association avec tout excipient approprié.

8. Composition pharmaceutique selon l'une des revendications 6 et 7, **caractérisée en ce qu'**elle contient un composé défini selon l'une des revendications **1** et **2** associé à un autre principe actif.

## Patentansprüche

1. Derivate von 3-Oxo-(2H)-1,2,4-triazin, welche der allgemeinen Formel I 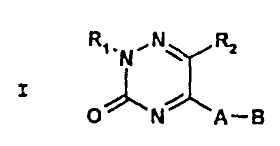 entsprechen, in der
- R1
Wasserstoff, jedoch nur, wenn A ein gegebenenfalls substituiertes Stickstoff-Atom ist,
eine lineare oder verzweigte (C₁-C₄)-Alkylgruppe,
eine Phenyl-(C₁-C₄)-alkylgruppe darstellt, wobei der Phenylkern gegebenenfalls durch ein oder mehrere (C₁-C₄)-Alkyl-, (C₁-C₃)-Alkoxy-, Halogen- oder Trifluormethyl-Gruppen substituiert ist;
- R2
Wasserstoff,
einen linearen oder verzweigten (C₁-C₄)-Alkylrest,
eine Phenyl- oder Phenyl-(C₁-C₄)-alkylgruppe darstellt, wobei der Phenylkern gegebenenfalls durch eine oder mehrere (C₁-C₄)-Alkyl-, (C₁-C₃)-Alkoxy-, Halogen- oder Trifluormethyl-Gruppen substituiert ist;
- A ein Sauerstoffatom oder ein gegebenenfalls substituiertes Stickstoff-Atom NR₃ darstellt;
- R₃ Wasserstoff oder eine Methylgruppe darstellt;
- B eine Gruppe der Art 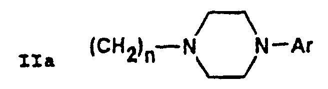 darstellt, in der Ar selbst eine aromatische Phenyl-, Pyridyl- oder Pyrimidyl-Struktur darstellt, die gegebenenfalls durch eine oder mehrere (C₁-C₃)-Alkyl-, (C₁-C₃)-Alkoxy-, Hydroxy-, Trifluormethyl- oder Halogengruppen substituiert ist, und n ganze Werte von 3 bis 5 annehmen kann; 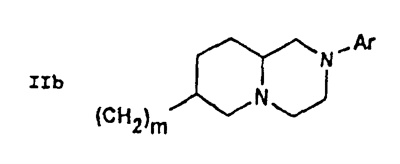 worin Ar wie in der Formel IIa definiert ist und m ganze Werte von 1 und 2 annehmen kann; 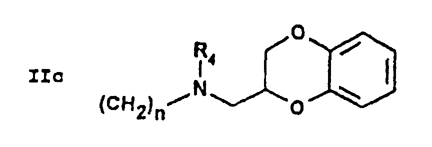 worin R₄ Wasserstoff oder eine (C₁-C₃)-Alkylgruppe darstellt und n ganze Werte von 3 bis 5 annehmen kann;
wobei die Verbindungen der allgemeinen Formel I in Form von sowohl racemischen Mischungen als auch verschiedenen reinen Enantiomeren oder Diastereoisomeren oder deren Mischungen vorliegen können,
sowie die therapeutisch annehmbaren Mineral- oder organischen Salze der Verbindungen der allgemeinen Formel I.

2. Verbindung nach Anspruch 1, **dadurch gekennzeichnet, dass** sie aus den folgenden Verbindungen ausgewählt ist:
2-Methyl-5-[4-(4-pyrimidin-2-ylpiperazin-1-yl)butoxy]-*2H*-[1,2,4]-triazin-3-on;
2-Methyl-5-[4-[4-(4-methylpyrimidin-2-yl)piperazin-1-yl]butoxy]-*2H*-[1,2,4]-triazin-3-onfumarat;
5-[4-[4-(4,6-Dimethylpyrimidin-2-yl)piperazin-1-yl]butoxy]-2-methyl-*2H*-[1,2,4]-triazin-3-onfumarat;
5-[4-[4-(4-Methoxypyrimidin-2-yl)piperazin-1-yl]butoxy]-2-methyl-*2H*-[1,2,4]-triazin-3-onfumarat;
5-[4-[4-(5-Methoxypyrimidin-2-yl)piperazin-1-yl]butoxy]-2-methyl-*2H*-[1,2,4]-triazin-3-on;
5-[4-[4-(4-Chlorpyrimidin-2-yl)piperazin-1-yl]butoxy]-2-methyl-*2H*-[1,2,4]-triazin-3-on;
5-[4-[4-(5-Fluorpyrimidin-2-yl)piperazin-1-yl]butoxy]-2-methyl-*2H*-[1,2,4]-triazin-3-on;
2-Propyl-5-[4-(4-pyrimidin-2-yl-piperazin-1-yl)butoxy]-*2H*-[1,2,4]-triazin-3-onfumarat;
2,6-Dimethyl-5-[4-[4-pyrimidin-2-ylpiperazin-1-yl)butoxy]*-2H*-[1,2,4]-triazin-3-on;
2-Methyl-6-phenyl-5-[4-(4-pyrimidin-2-ylpiperazin-1-yl)butoxy]*-2H*-[1,2,4]-triazin-3-on;
2-Methyl-6-benzyl-5-[4-(4-pyrimidin-2-ylpiperazin-1-yl)butoxy]-*2H*-[1,2,4]-triazin-3-on;
2-Benzyl-5-[4-(4-pyrimidin-2-ylpiperazin-1-yl)butoxy]-*2H*-[1,2,4]-triazin-3-on;
2-Methyl-5-[3-(4-pyrimidin-2-ylpiperazin-1-yl]propoxy]-*2H*-[1,2,4]-triazin-3-onfumarat;
2-Methyl-5-[5-(4-pyrimidin-2-ylpiperazin-1-yl)pentyloxy]-*2H*-[1,2,4]-triazin-3-on;
5-[4-[4-(3-Methoxypyridin-2-yl)piperazin-1-yl]butoxy]-2-methyl-*2H*-[1,2,4]-triazin-3-onfumarat;
5-[4-[4-(3-Chlorphenyl)piperazin-1-yl]butoxy]-2-methyl-*2H*-[1,2,4]-triazin-3-onfumarat;
5-[4-[4-(5-Chlor-2-methoxyphenyl)piperazin-1-yl]butoxy]-2-methyl-*2H*-[1,2,4]-triazin-3-onfumarat;
4-[4-[4-(2-Methyl-3-oxo-2,3-dihydro-[1,2,4]-triazin-5-yloxy)butyl]piperazin-1-yl]benzonitril;
5-[4-[4-(5-Chlor-2-methoxyphenyl)piperazin-1-yl]butoxy]-2-methyl-*2H*-[1,2,4]-triazin-3-onfumarat;
5-[4-[(2,3-Dihydrobenzo[1,4]dioxin-2-ylmethyl)amino]butoxy]-2-methyl-*2H*-[1,2,4]-triazin-3-onfumarat;
(R)-5-[4-[(2,3-Dihydrobenzo[1,4]dioxin-2-ylmethyl]amino]butoxy]-2-methyl-*2H*-[1,2,4]-triazin-3-onfumarat;
(S)-5-[4-[(2,3-Dihydrobenzo[1,4]dioxin-2-ylmethyl]amino]butoxy]-2-methyl-*2H*-[1,2,4]-triazin-3-onfumarat;
trans-2-Methyl-5-(2-pyrimidin-2-yloctahydropyrido[1,2-a]pyrazin-7-ylmethoxy)-*2H*-[1,2,4]-triazin-3-on;
trans-(-)-2-Methyl-5-[2-(2-pyrimidin-2-yloctahydropyrido[1,2-a]pyrazin-7-yl)-ethoxy]-*2H*-[1,2,4]-triazin-3-on;
trans-(+)-2-Methyl-5-[2-(2-pyrimidin-2-yloctahydropyrido[1,2-a]pyrazin-7-ylethoxy]-*2H*-[1,2,4]-triazin-3-on;
2-Methyl-5-[4-(4-pyrimidin-2-ylpiperazin-1-yl)butylamino]-*2H*-[1,2,4]-triazin-3-on.

3. Verfahren zur Herstellung von chemischen Verbindungen nach Anspruch 1 und 2, **dadurch gekennzeichnet, dass** man:
ein Derivat der allgemeinen Formel III 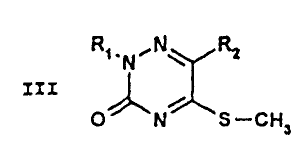 mit einem Alkohol B-OH IV oder einem Amin BNHR₃ V, wobei R₁, R₂, R₃ und B wie in Anspruch 1 definiert sind, in Anwesenheit von Natriumhydrid oder Kaliumtertiärbutanolat in Dioxan, Tetrahydrofuran oder Toluol behandelt.

4. Verbindungen, definiert nach einem der Ansprüche 1 und 2, als Medikamente, die bei der Behandlung von Krankheiten verwendbar sind, welche Agonisten des Rezeptors 5HT_{1A} erfordern.

5. Verbindungen, definiert nach einem der Ansprüche 1 und 2, als Medikamente, die beispielsweise für die Behandlung von Angst, Depression, Schmerz, Neurodegeneration, Schizophrenie, Alzheimer-Krankheit, Schlafstörungen, die Regulierung der Nahrungsaufnahme, die Regulierung der Magensekretion, die Behandlung von vaskulären, kardiovaskulären und cerebrovaskulären Störungen nützlich sind.

6. Pharmazeutische Zusammensetzung, **dadurch gekennzeichnet, dass** sie als Wirkstoff eine Verbindung, definiert gemäß einem der Ansprüche 1 und 2, enthält.

7. Pharmazeutische Zusammensetzung nach Anspruch 6, **dadurch gekennzeichnet, dass** sie eine Verbindung, definiert gemäß einem der Ansprüche 1 und 2, in Verbindung mit irgendeinem geeigneten Hilfsmittel enthält.

8. Pharmazeutische Zusammensetzung nach einem der Ansprüche 6 und 7, **dadurch gekennzeichnet, dass** sie eine Verbindung, definiert gemäß einem der Ansprüche 1 und 2, in Verbindung mit einem anderen Wirkstoff enthält.

## Claims

1. 3-Oxo-(*2H*)-1,2,4-triazine derivatives corresponding to the general formula I 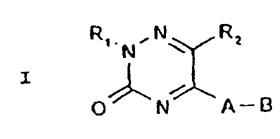 in which
- R₁ represents:
• hydrogen, solely when A is an optionally substituted nitrogen atom
• a linear or branched C₁-C₄ alkyl group
• a C₁-C₄ phenylalkyl group, the phenyl nucleus optionally being substituted by one or more C₁-C₄ alkyl, C₁-C₃ alkoxy, halogen or trifluoromethyl groups,
- R₂ represents:
• hydrogen
• a linear or branched C₁-C₄ alkyl radical
• a C₁-C₄ phenyl or phenylalkyl group, the phenyl nucleus optionally being substituted by one or more C₁-C₄ alkyl, C₁-C₃ alkoxy, halogen or trifluoromethyl groups,
- A represents an oxygen atom or an optionally substituted nitrogen atom NR₃,
- R₃ represents hydrogen or a methyl group,
- B represents a group of type 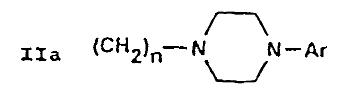 in which Ar itself represents a phenyl, pyridyl or pyrimidyl aromatic structure, optionally substituted by one or more groups C₁-C₃ alkyl, C₁-C₃ alkoxy, hydroxyl, trifluoromethyl or halogen groups, and n can take the integral values from 3 to 5, 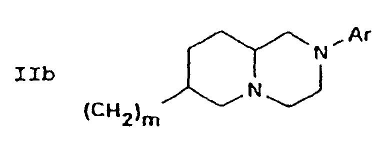 in which Ar is as defined in the formula IIa and m can take the integral values 1 and 2, 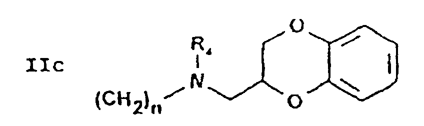 in which R₄ represents hydrogen or a C₁-C₃ alkyl group and n can take the integral values from 3 to 5.
The compounds of general formula I exist in the form both of racemic mixtures and of various pure enantiomers or diastereoisomers or of their mixtures, and the therapeutically acceptable inorganic or organic salts of the compounds of general formula I.

2. Compound according to Claim 1, **characterized in that** it is chosen from the following compounds:
* 2-Methyl-5-[4-(4-pyrimidin-2-yl-piperazin-1-yl)butoxy]-2*H*-[1,2,4]triazin-3-one
* 2-Methyl-5-[4-[4-(4-methylpyrimidin-2-yl)-piperazin-1-yl]butoxy]-2*H*-[1,2,4]triazin-3-one fumarate
* 5-[4-[4-(4,6-Dimethylpyrimidin-2-yl)piperazin-1-yl]butoxy]-2-methyl-2*H*-[1,2,4]triazin-3-one fumarate
* 5-[4-[4-(4-Methoxypyrimidin-2-yl)piperazin-1-yl]butoxy]-2-methyl-2*H*-[1,2,4]triazin-3-one fumarate
* 5-[4-[4-(5-Methoxypyrimidin-2-yl)piperazin-1-yl]butoxy]-2-methyl-2*H*-[1,2,4]triazin-3-one
* 5-[4-[4-(4-Chloropyrimidin-2-yl)piperazin-1-yl]butoxy]-2-methyl-2*H*-[1,2,4]triazin-3-one
* 5-[4-[4-(5-Fluoropyrimidin-2-yl)piperazin-1-yl]butoxy]-2-methyl-2*H*-[1,2,4]triazin-3-one
* 2-Propyl-5-[4-(4-pyrimidin-2-yl-piperazin-1-yl)butoxy]-2*H*-[1,2,4]triazin-3-one fumarate
* 2,6-Dimethyl-5-[4-(4-pyrimidin-2-yl-piperazin-1-yl)butoxy]-2*H*-[1,2,4]triazin-3-one
* 2-Methyl-6-phenyl-5-[4-(4-pyrimidin-2-yl-piperazin-1-yl)butoxy]-2*H*-[1,2,4]triazin-3-one
* 2-Methyl-6-benzyl-5-[4-(4-pyrimidin-2-yl-piperazin-1-yl)butoxy]-2*H*-[1,2,4]triazin-3-one
* 2-Benzyl-5-[4-(4-pyrimidin-2-yl-piperazin-1-yl)butoxy]-2*H*-[1,2,4]triazin-3-one
* 2-Methyl-5-[3-(4-pyrimidin-2-yl-piperazin-1-yl)propoxy]-2*H*-[1,2,4]triazin-3-one fumarate
* 2-Methyl-5-[5-(4-pyrimidin-2-yl-piperazin-1-yl)pentyloxy]-2*H*-[1,2,4]triazin-3-one
* 5-[4-[4-(3-Methoxypyridin-2-yl)piperazin-1-yl]butoxy]-2-methyl-2*H*-[1,2,4]triazin-3-one fumarate
* 5-[4-[4-(3-Chlorophenyl)piperazin-1-yl]-butoxy]-2-methyl-2*H*-[1,2,4]triazin-3-one fumarate
* 5-[4-[4-(5-Chloro-2-methoxyphenyl)piperazin-1-yl]butoxy]-2-methyl-2*H*-[1,2,4]triazin-3-one fumarate
* 4-[4-[4-(2-Methyl-3-oxo-2,3-dihydro-[1,2,4]-triazin-5-yloxy)butyl]piperazin-1-yl]benzonitrile
* 5-[4-[4-(5-Chloro-2-methoxyphenyl)piperazin-1-yl]butoxy]-2-methyl-2*H*-[1,2,4]triazin-3-one fumarate
* 5-[4-[(2,3-Dihydrobenzo[1,4]dioxin-2-yl-methyl)amino]butoxy]-2-methyl-2*H*-[1,2,4]-triazin-3-one fumarate
* (R)-5-[4-[(2,3-Dihydrobenzo[1,4]dioxin-2-yl-methyl)amino]butoxy]-2-methyl-2*H*-[1,2,4]-triazin-3-one fumarate
* (S)-5-[4-[(2,3-Dihydrobenzo[1,4]dioxin-2-yl-methyl)amino]butoxy]-2-methyl-2*H*-[1,2,4]-triazin-3-one fumarate
* trans-2-Methyl-5-(2-pyrimidin-2-yl-octahydropyrido[1,2-a]pyrazin-7-ylmethoxy)-2*H-*[1,2,4]-triazin-3-one
* trans-(-)-2-Methyl-5-[2-(2-pyrimidin-2-yl-octahydropyrido[1,2-a]pyrazin-7-yl)ethoxy]-2*H*-[1,2,4]triazin-3-one
* trans-(+)-2-Methyl-5-[2-(2-pyrimidin-2-yl-octahydropyrido[1,2-a]pyrazin-7-yl)ethoxy]-2*H*-[1,2,4]triazin-3-one
* 2-Methyl-5-[4-(4-pyrimidin-2-yl-piperazin-1-yl)butylamino]-2*H*-[1,2,4]triazin-3-one.

3. Process for the preparation of the chemical compounds according to Claims 1 and 2, **characterized:**
**in that** a derivative of general formula III 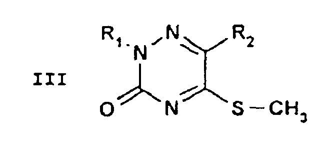 is treated with an alcohol B-OH IV or an amine BNHR₃ V, R₁, R₂, R₃ and B being as defined in Claim 1, in the presence of sodium hydride or potassium tert-butoxide in dioxane, tetrahydrofuran or toluene.

4. As medicaments which can be used in the treatment of diseases requiring agonists of 5-HT_{1A} receptors, the compounds defined according to either of Claims 1 and 2.

5. As medicaments of use, for example, for the treatment of anxiety, depression, pain, neuro-degeneration, schizophrenia, Alzheimer's disease and sleep disorders, the regulation of food intake, the regulation of gastric secretion and the treatment of vascular, cardiovascular and cerebrovascular disorders, the compounds defined according to either of Claims 1 and 2.

6. Pharmaceutical composition, **characterized in that** it comprises, as active principle, a compound defined according to either of Claims 1 and 2.

7. Pharmaceutical composition according to Claim 6, **characterized in that** it comprises a compound defined according to either of Claims 1 and 2 in combination with any appropriate excipient.

8. Pharmaceutical composition according to either of Claims 6 and 7, **characterized in that** it comprises a compound defined according to either of Claims 1 and 2 in combination with another active principle.
